(19) Europäisches Patentamt
European Patent Office
Office européen des brevets

(11) Publication number: **0 163 151**
**A1**

(12) # EUROPEAN PATENT APPLICATION

(21) Application number: 85105254.8

(22) Date of filing: 26.04.85

(51) Int. Cl.⁴: **A 61 L 15/00**
C 08 F 8/30

(30) Priority: 27.04.84 US 604709

(43) Date of publication of application:
04.12.85 Bulletin 85/49

(84) Designated Contracting States:
BE DE FR GB IT LU NL SE

(71) Applicant: PERSONAL PRODUCTS COMPANY
Van Liew Avenue
Milltown New Jersey 08850(US)

(72) Inventor: Dabi, Shmuel
218 North Second Avenue
Highland Park, N.J. 08904(US)

(74) Representative: Groening, Hans Wilhelm, Dipl.-Ing.
Siebertstrasse 4 Postfach 860 340
D-8000 München 86(DE)

(54) **Crosslinked carboxyl polyelectrolytes and method of making same.**

(57) Crosslinked water absorbent carboxylic polyelectrolytes and articles made therefrom are provided. The polyelectrolytes are crosslinked using a di- or tri- functional aziridine crosslinking agent.

EP 0 163 151 A1

# CROSS-LINKED CARBOXYL POLYELECTROLYTES AND METHOD OF MAKING SAME

## Background of the Invention

This invention relates to highly absorbent and retentive materials for absorbing aqueous based fluids and more particularly for absorbing body fluids in such products as sanitary napkins, diapers, bandages and the like. Specifically, this invention is related to compositions and methods for preparing crosslinked polyelectrolytes capable of absorbing and retaining many times their weight of such aqueous fluids.

Highly absorbent crosslinked polyelectrolytes and methods of preparing the same are already known. U.S. Patent Nos. 3,669,103 and 3,670,731 teach use of these materials in diapers and dressings. U.S. Patent Nos. 2,988,539; 3,393,168; 3,514,419 and 3,557,067 teach methods of making such absorbents and in particular are related to water swellable crosslinked carboxylic copolymers that are either crosslinked during copolymerization or crosslinked after polymerization and then neutralized to result in pendant ionic moieties capable of imparting water retention properties to the finished material. Additionally polyelectrolytes have been prepared which are cured or crosslinked employing epihalohydrine. For example, U.S. Patent No. 3,980,663 employ epihalohydrines but has been found to be unacceptably slow and inefficient as a method for crosslinking such polymers.

In U.S. Patent No. 4,076,673 an improvement in the rate and efficiency of curing polyelectrolytes has been accomplished by employing as a crosslinking agent a polyamidepolyamine epihalohydrine adduct of the kind commercially available from Hercules, Incorporated and sold by them under the Trademark Polycup®. A still further improvement in the choice of crosslinking agent is disclosed in U.S. Patent No. 4,310,593 which teaches the use of monomeric amine epihalohydrine as a crosslinker which is said to have greater efficiency, longer shelf life and the convenience of being capable of shipping as a concentrate.

The above disclosures notwithstanding, there is still a need for improvement in crosslinked polyelectrolyte technology and in particular there is a need to improve the efficiency of the crosslinking reaction, the shelf life of the crosslinker and its convenience in use.

## Summary of the Invention

It has now been discovered that a crosslinker may be employed to cure or crosslink polyelectrolytes which exhibits significantly greater efficiency, faster reaction rate and greater shelf life without sacrificing any of the advantages of prior suggested processes.

In particular it has been discovered that these desirable attributes may be achieved by employing in such compositions and methods a crosslinking agent which is a water soluble, relatively low molecular weight compound having at least two 1-aziridinyl groups bonded thereto. Preferably, such groups have the general formula:

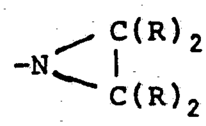

wherein each R group is independently selected from the group consisting of H, alkyl having from one to three carbon atoms, or alkylene having from one to three carbon atoms. Preferably the compound has a molecular weight of less than 1000. Of choice the functional groups are bonded to aliphatic or substituted aliphatic groups sufficiently small enough to maintain the compound soluble in water. The crosslinkers of choice are di- and tri-functional aziridine several of which are commercially available.

It has been found that employment of the crosslinking agents of this invention results in the ability to use only minimal amounts of crosslinking agents, that the curing rates of these agents are demonstratably faster than prior agents and that the shelf life of these agents, notwithstanding their higher reactivity, are substantially longer than prior agents.

## Detailed Description of the Invention

The carboxylic polyelectrolytes capable of being crosslinked in accordance with the teachings of this invention are well-known and are described in detail in U.S. Patent No. 4,310,593 which is incorporated herein by reference. The essence of usable polyelectrolytes is that they comprise, at least in the salt form, sufficient carboxylate moieties to render them water soluble. Usable polymers, capable of being prepared from readily available monomers and converted into their salt form include for example, acrylic acid-acrylate copolymers; acrylic acid-acrylamide

copolymers; acrylic acid-olefin copolymers; polyacrylic acid; acrylic acid-vinyl aromatic copolymers; acrylic acid-styrene sulfonic acid copolymers; acrylic acid-vinyl ether copolymers; acrylic acid vinyl acetate copolymers, acrylic acid-vinyl alcohol copolymers; copolymers of methacrylic acid with all of the above comonomers; copolymers of maleic acid, fumaric acid and their esters with all of the above comonomers; copolymers of maleic anhydride with all of the above comonomers.

Generally described, the crosslinking agents of this invention are low molecular weight, water soluble compounds having at least two 1-aziridinyl groups bonded thereto which groups preferably have the general formula:

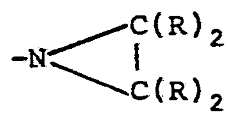

wherein the R groups may be independently selected from the group comprising H, alkyl having from one to three carbon atoms or alkenyl having from two to three carbon atoms. The functional groups are preferably bonded to an aliphatic chain or a substituted aliphatic chain with the essential criterion that such chains be small enough to insure that the compound is water soluble. Preferably the compound has a molecular weight of less than 1000. Such aliphatic or substituted aliphatic chains may include olefinic groups from 2 to 12 carbon atoms; substituted olefinic groups such as olefinic hydroxides, e.g., butylenehydroxide or butylenedihydroxide; mercaptans of olefins such as mercaptobutylene; ethers of aliphatic compounds such as diethylene glycol or triethylene glycol; esters of aliphatic compounds such as triglycerides or esters of trimethylpropane and pentaerythritol.

Several such compounds are already commercially available and it will be understood by one skilled in the art that a great many variations of these commercially available compounds can be synthesized while still conforming to the general description given above. A particularly effective group of compounds are the triaziridines based on trimethylolpropane tripropionate adducts having the formula:

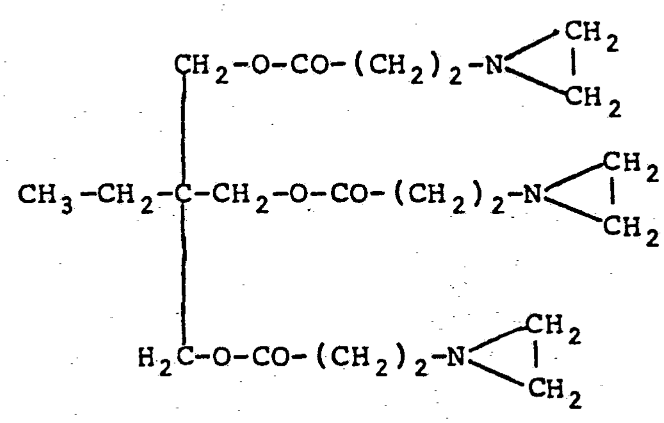

and sold by the Aceto Chemical Company under the trade name TAZ.

Another effective compound, based on pentaerythritol tripropionate adduct, has the formula:

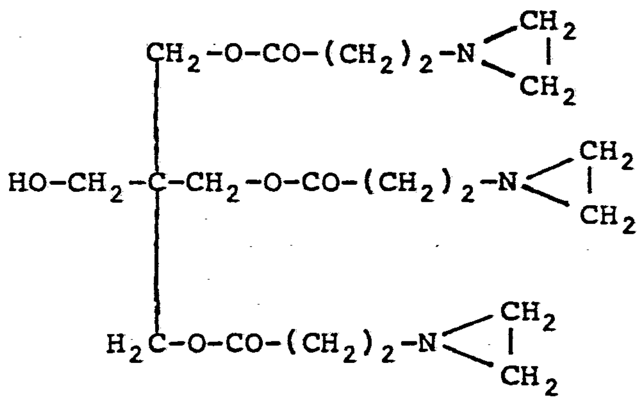

and is sold by Aceto Chemical Company under the trade name TAZO. Similar materials conforming to the general description given above are available from Cordova Chemical Company under the trade name XAMA. Additionally, other polyfunctional aziridines that have triazine or phosphate backbones are also available. Such are, for example, tris(1-aziridinyl)phosphine oxide, tris(1-aziridinyl)-phosphine sulfide; 2,4,6,trisaziridinyl-s-triazine.

The reaction of the functional group of the aziridine with the carboxyl group of a carboxylic polyelectrolyte proceeds rapidly at temperatures of from room temperature or less to about 150°C with, of course, increasing reaction rate the highest temperatures. The reaction proceeds through ring opening as follows:

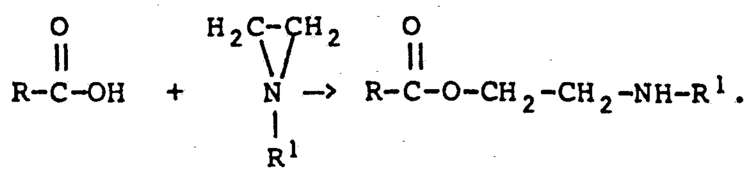

Crosslinking takes place when a polyfunctional aziridine molecule reacts as above with carboxyl groups of adjacent polyelectrolytes to form bridges between these molecules.

In the general method of carrying out this invention, a solution is prepared comprising from 5 to 60% by weight of a carboxylic polyelectrolyte, e.g., polyacrylic acid or salts thereof having molecular weights which may vary from 10,000 to 4,000,000 or more. Preferably such molecular weights should range from 10,000 to 1,000,000. As used in this connection, the term "molecular weight" is meant to denote the weight average molecular weight of the polymer. The choice of molecular weight for the polyelectrolyte may vary over this wide range depending on the desired properties of the finished crosslinked product. For example, extremely low weight polyelectrolytes will go into solution easily and hence highly concentrated solutions may be prepared which will require less drying to produce the final product. Low weight polyelectrolytes however require a higher ratio of crosslinking agent to insolubilize. On the other hand, extremely high molecular weight polyelectrolytes form very viscous solutions which are difficult to handle in their pre-crosslinked form but require lower concentration of crosslinking agent to insolubilize.

The solvent of choice is water although other solvents such as alcohol or mixtures of water and alcohol may be employed.

The solution may be maintained in the acid state or may be neutralized to as high a pH as 12 or more utilizing a strong base such as sodium hydroxide. Preferably the reaction is best controlled when preparing a relatively neutral solution in the range of from 5 to 10 pH and still more preferably in the range of from 6 to 8.

The polyfunctional aziridine is dissolved into the solution at a concentration which may vary from about 0.2 to about 20% by weight, based on the weight of the carboxylic polyelectrolyte. Preferably, the concentration should range from 0.5 to 15% and still more preferably from 1 to 10%. For a given polyelectrolyte, too low a concentration of aziridine will result in a failure to render the polyelectrolyte insoluble. On the other hand, too high a concentration of aziridine will result in a crosslinked product which exhibits relatively low swelling and hence low absorption capacity. These properties also vary with the moleclar weight of the uncrosslinked polymer wherein a greater concentration of crosslinking agent is required to insolubilize a low molecular weight polyelectrolyte and a lesser quantity of crosslinker may be employed with higher molecular weight polyelectrolytes. In general, to obtain best absorption properties, the minimum quality of crosslinking agent capable of insolubilizing the polyelectrolyte should be employed.

The solution may be cast onto a release substrate to produce a highly absorbent film upon drying. The film may then be crushed to form an absorbent powder, the common form of highly absorbent materials suggested for use in

such body fluid absorbent products as diapers, sanitary napkins, tampons, dressings or the like.

A particularly useful material may be produced by employing, as the neutralizing base, a carbonate compound such as $Na_2CO_3$, $K_2CO_3$ or ammonium bicarbonate. The neutralization of the carboxylic polyelectrolyte will release carbon dioxide gas and foam the mixture which, on drying, produces a cellular, foam-like material having high volume and enhanced absorbency. Ordinarily, it would be difficult to use such a system to foam the polymers as the cells tend to collapse too quickly. However, because of the rapidity of reaction with the crosslinking agents of this invention this is now possible.

Another method of utilizing the compositions and methods of the invention is to spray the solution of polyelectrolytes and crosslinkers onto the surface of a substrate such a tissue which when dried will exhibit good absorption properties. Alternatively, the solution may be applied by padding or saturating a substrate such as a pad of cellulose fibers or the like which then may be dried, with the highly absorbent crosslinked polyelectrolyte distributed throughout the pad. Vacuum suction means can be employed to control the solution add-on as is currently employed for producing suction bonded nonwoven fabrics. The dried crosslinked polyelectrolyte adheres to most surfaces with sufficient tenacity to preclude problems such as sifting or dislocation typically encountered when attempting to combine absorbent powders with nonwoven fabrics or pads of cellulosic materials.

Example 1

A composition is prepared by mixing a 25%, by weight, aqueous solution of poly (sodium acrylate) having a

molecular weight of 100,000 with a sufficient quantity of 50% by weight of aqueous sodium hydroxide to neutralize to a pH of from 7 to 8. The triaziridine based on trimethylolpropane tripropionate adduct, TAZ, and obtained from the Aceto Chemical Company, is dissolved into the solution in various concentrations, as are set out in Table 1 below in percent by weight, based on the weight of the poly(sodium acrylate). A film of the solution, 30 mils thick is cast onto silicon coated paper and dried in an oven at 110°C for 30 minutes. The films are peeled from the paper and ground to 20 mesh powders. The weighed powders are saturated with test fluids (deionized water and a 1% by weight aqueous NaCl solution), excess fluid is absorbed with paper towels and the weight gain of water is measured as the Free Absorbency. The results are reported below in Table 1.

Table 1

| Crosslinker (TAZ), % | Free Absorbency, g/g | |
|---|---|---|
| | Water | 1% NaCl |
| 0.5 | soluble | -- |
| 1.2 | 75 | 12 |
| 2.0 | 128 | 22 |
| 4.0 | 70 | 10 |
| 7.0 | 10 | -- |

As can be seen from the above table, this relatively low molecular weight polyelectrolyte reached an optimum Free Absorbency value at approximately 2% crosslinker concentration and thereafter exhibited reduced absorbency.

Example 2

The procedure of Example 1 is followed with the exception that a 200,000 molecular weight poly (sodium acrylate) was employed. The crosslinker used for this Example 2 is the triaziridine based on pentaerythritol tripropionate adduct, TAZ-0, sold by the Aceto Chemical Company. The results are shown in Table 2 below.

Table 2

| Crosslinker (TAZ-0), % | Free Absorbency, g/g | |
|---|---|---|
| | Water | 1% NaCl |
| 8 | 60 | -- |
| 4 | 70 | -- |
| 2 | 140 | 35 |
| 1 | 330 | 45 |
| 0.6 | - soluble | - |

As can be seen, this relatively high molecular weight polyelectrolyte exhibited optimum absorbency at about 1% crosslinker concentration.

Example 3

The procedure of Example 1 is repeated with the exception that the polyelectrolyte employed was poly(acrylic acid) neutralized with sodium hydroxide and having varying molecular weights as set out in Table 3 below and the crosslinking agent employed is TAZ-0 at a constant concentration of 1%. As in Example 1 a 25% solution of the polyelectrolyte is employed with the exception that, for the 400,000 molecular weight polyacrylic acid, a 12.5% solution is used. The results are reported below in Table 3.

Table 3

| Poly(acrylic acid) Molecular Weight | Free Absorbency (g/g) Water | 1% NaCl |
|---|---|---|
| 25,000 | - soluble - | |
| 60,000 | 130 | 29 |
| 200,000 | 360 | 40 |
| 400,000 | 435 | 43 |

As can be seen from this example, for the constant value of crosslinker concentration, absorbency increased with increasing molecular weight.

Example 4

A crosslinked absorbent polyelectrolyte foam is prepared. A mixture of three grams of sodium hydroxide, three grams of potassium carbonate, and one gram of ammonium bicarbonate are dissolved in 80 grams of water and one gram of the TAZ triaziridine crosslinking agent. The solution is poured on 10 grams of 450,000 molecular weight polyacrylic acid powder obtained from Polysciences, Inc. and hand mixed thoroughtly for three minutes. The mixture has a dough-like consistency due to the carbon dioxide released from the carbonate compounds. The mixture is heated for 30 seconds in a microwave oven and then kept for 15 minutes in a hot air oven at 120°C. The resulting dry material is fluffy and cellular and demonstrates a very high absorption rate. The material is crushed manually and the free absorbency is measured as described above. The material is found to absorb 480 grams per gram of deionized water and 55 grams per gram of one percent, by weight, sodium chloride aqueous solution.

Example 5

A crosslinked polyelectrolyte absorbent film is produced. A 25 percent aqueous solution of polyacrylic acid having a molecular weight of 100,000 and obtained from the North Chemical Inc. Company in the quantity of 26.4 grams was neutralized with four grams of potassium carbonate and two grams of ammonium bicarbonate. Two drops of a surfactant, pluronic L-62, was added to break up the foam. The crosslinker, TAZ triaziridine, was mixed into the solution and a film was cast on a silicone coated paper. After 15 minutes, at 110°C, a strong but brittle film was obtained that swelled instantly when wetted by water. The film was crushed in a mortar and the powder had a free absorbency of 128 grams per gram of water and 22 grams per gram of one percent sodium chloride solution. Measurements for absorption retention under pressure were taken using the gravimetric absorbency tester (GAT) instrument, described in U.S. Patent No. 4,357,827. The absorption using this instrument was found to be 20.8 grams per gram with a retention of 14.8 grams per gram using a 1%, by weight, aqueous sodium chloride test fluid.

Example 6

The formulation of Example 5 was diluted with water to give a 10 percent by weight solid solution. Solution was sprayed onto a non-woven fabric of cellulosic fibers while vacuum was applied on the opposite side of the fabric to suck the excess fluid through the fabric. The solution was also sprayed onto a tissue paper. Both impregnated substrates were dried for 10 minutes at 110°C. Similar samples were prepared and were dried overnight at room temperature. The absorption characteristics of these impregnated substrates varied with the amount of solution applied thereto but all exhibited enhanced absorbency.

What is Claimed Is:

1. In a curable composition, useful to form absorbent articles of a carboxylic polyelectrolyte, which composition comprises a solution of:

(1) a solvent selected from the group consisting of water, lower alcohols, and mixtures thereof,

(2) about 5 to 60% by weight of a carboxylic polyelectrolyte or mixtures thereof;

the improvement which comprises employing:

(3) a crosslinking agent comprising a water soluble, relatively low molecular weight compound having at least two 1-aziridinyl groups bonded thereto;

said crosslinking agent present in an amount sufficient to cure said polyelectrolyte into a water absorbent article.

2. The composition of claim 1 wherein said 1-aziridinyl groups have the formula:

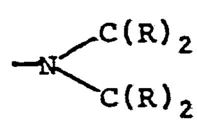

wherein each R group is independently selected from the group consisting of H, alkyl having from one to three carbon atoms, or alkenyl having from two to three carbon atoms.

3. The composition of claim 1 wherein said compound has a molecular weight of less than 1000.

4. The composition of claim 1 wherein said functional groups are bonded to aliphatic or substituted aliphatic groups which are sufficiently small enough to maintain the compound soluble in water.

5. The composition of claim 1 wherein said compound is a di-functional aziridine.

6. The composition of claim 1 wherein said compound is a tri-functional aziridine.

7. The composition of claim 6 wherein said tri-functional aziridine is:

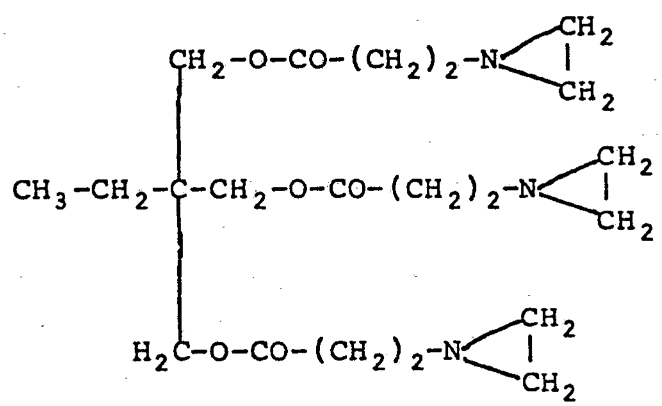

8. The composition of claim 6 wherein said tri-functional aziridine is:

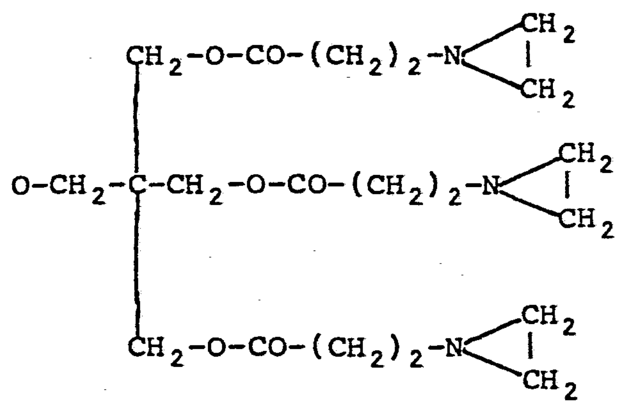

9. The composition of claim 1 wherein said polyelectrolyte has a molecular weight of from 10,000 to 4,000,000.

10. The composition of claim 1 wherein said compound is present in an amount of from about 0.2% to about 20%, by weight, based on the weight of the polyelectrolyte.

11. The composition of claim 1 wherein said compound is present in an amount of from about 0.5 to about 15% by weight based on the weight of the polyelectrolyte.

12. The composition of claim 1 wherein the compound is present in an amount of from about 1% to about 10% by weight based on the weight of the polyelectrolyte.

13. A method of preparing a water absorbent polyelectrolyte comprising the steps of:

forming a composition by combining 1) a solvent selected from the group consisting of water, lower alcohols, and mixtures thereof with 2) about 5 to 60% by weight of carboxylic polyelectrolyte or mixtures thereof and 3) a crosslinking agent comprising a water soluble, relatively low molecular weight compound having at least two

1-aziridinyl groups bonded thereto;

crosslinking said carboxylic polyelectrolyte with said crosslinking agent and drying the resultant product.

14. The method of claim 13 wherein said 1-aziridinyl groups have the formula:

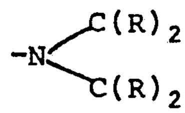

wherein each R group is independently selected from the group consisting of H, alkyl having from one to three carbon atoms, or alkenyl having from two to three carbon atoms.

15. The method of claim 13 for preparing said absorbent polyelectrolyte in the form of a film comprising spreading said composition on an impervious substrate and then crosslinking and drying said composition.

16. The method of claim 13 for preparing said water absorbent polyelectrolyte as a foam comprising including in said composition a neutralizing base capable of releasing gas upon neutralizing.

17. The method of claim 16 wherein said neutralizing base is a carbonate compound.

18. The method of claim 17 wherein said carbonate compound is selected from the group consisting of sodium carbonate, potassium carbonate, or ammonium bicarbonate.

European Patent Office

# EUROPEAN SEARCH REPORT

0163151
Application number

EP 85 10 5254

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| Y | EP-A-0 083 022 (SEITETSU KAGAKU CO., LTD.) * Claims 1-8,11; page 10, line 11 - page 12, line 22 * --- | 1-12 | A 61 L 15/00<br>C 08 F 8/30 |
| Y | FR-A-2 525 121 (NIPPON SHOKUBAI KAGAKU KOGYO CO., LTD.) * Claims 1,2,8,9; page 4, lines 12-33 * --- | 1-12 | |
| A | US-A-4 018 951 (J.R. GROSS) * Claim 1 * ----- | 13,15 | |
| | | | TECHNICAL FIELDS SEARCHED (Int. Cl.4) |
| | | | A 61 L<br>C 08 F |

The present search report has been drawn up for all claims

| Place of search | Date of completion of the search | Examiner |
|---|---|---|
| THE HAGUE | 10-09-1985 | PERMENTIER W.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82